# EUROPEAN PATENT SPECIFICATION

(11) **EP 4 522 284 B1**
(45) Date of publication and mention of the grant of the patent: **25.02.2026**
(21) Application number: 23720766.7
(22) Date of filing: 13.04.2023
(51) Int. Cl.: A62B 18/02, A62B 18/08, A62B 18/00

(54) **VENTILATED PROTECTION DEVICE FOR THE RESPIRATORY TRACT**
BELÜFTETE ATEMSCHUTZVORRICHTUNG
DISPOSITIF DE PROTECTION VENTILÉ POUR LES VOIES RESPIRATOIRES

(30) Priority: 10.05.2022 IT 202200009635
(43) Date of publication of application: 19.03.2025
(73) Proprietor: Marino, Silvio, 54011 Aulla (MS) (IT)
(72) Inventor: Marino, Silvio, 54011 Aulla (MS) (IT)
(74) Representative: Sergio, Stefano
(86) International application number: PCT/EP2023/025173
(87) International publication number: WO 2023/217409

(56) References cited:
- KR-A- 20160 129 562
- US-A- 4 671 268
- US-A1- 2020 406 069
- US-A1- 2022 080 232
- US-B2- 9 248 248

## Description

This invention refers to a respiratory tract protection device equipped with a breathing aid by fan.

Various devices for protecting the airways from fine dust, fumes and liquid mists (aerosols) are commonly known. Aerosols and fine dust particles are some of the most hazardous health risks, as unfortunately we have been able to verify with the spread of the well-known Covid-19 virus, being almost invisible in breathable air. Filter masks against these particles offer protection from these dangers and are divided into three protection classes: FFP1, FFP2 and FFP3.

These masks are, moreover, prescribed in the workplace where the so-called occupational exposure limit or OEL value is exceeded. This indicates the maximum permissible concentration of dust, smoke or aerosols in the breathing air, which does not cause harm to health. When this value is exceeded, the use of filter masks becomes mandatory.

With regard to surgical masks, these have the purpose of preventing the wearer from contaminating the environment, as they limit the transmission of infectious agents and fall within the scope of medical devices referred to in Legislative Decree 24 February 1997, no. 46 and successive modifications.

They are used in hospitals and in places where assistance is provided to patients (for example, care homes, clinics, etc.) and, to be considered safe, they must be produced in compliance with the technical standard UNI EN 14683:2019 which provides features and test methods to evaluate resistance to liquid splashes, breathability, bacterial filtration efficiency, etc.

FFP1 masks have a reduced filtering capacity from the outside towards the wearer, of about 20%, mainly due to poor adherence to the face.

Traditional masks, however, take the air from the front and this is particularly disadvantageous as dust, fumes, aerosols are normally concentrated in the front part of the face as the operator is normally turned towards the work area and this also happens when two or more people talk to each other.

Another drawback is the fact that inside the mask there is poor air circulation which can have negative consequences for the person's health.

The inventor of the present invention has already filed, on 15/10/2020, the Patent application for Industrial Invention No. 102020000024334 "Protection device for the respiratory tract", in order to provide a protection device for the respiratory tract that is more efficacious than the existing ones in terms of protection from health-damaging agents and, at the same time, is able to improve the quality of air taken from outside.

A known respiratory protection device is disclosed in US 2020/406069 A1.

With the present invention, the applicant intends to provide a respiratory protection device which is capable of increasing ventilation inside the device itself and of diffusing inside it perfumed, balsamic or medicinal air-soluble substances.

A respiratory protection device is therefore provided according to the characteristics of the attached claim 1.

The invention will be better explained through the following description which shows the preferred embodiments made with the aid of the attached figures which respectively illustrate:

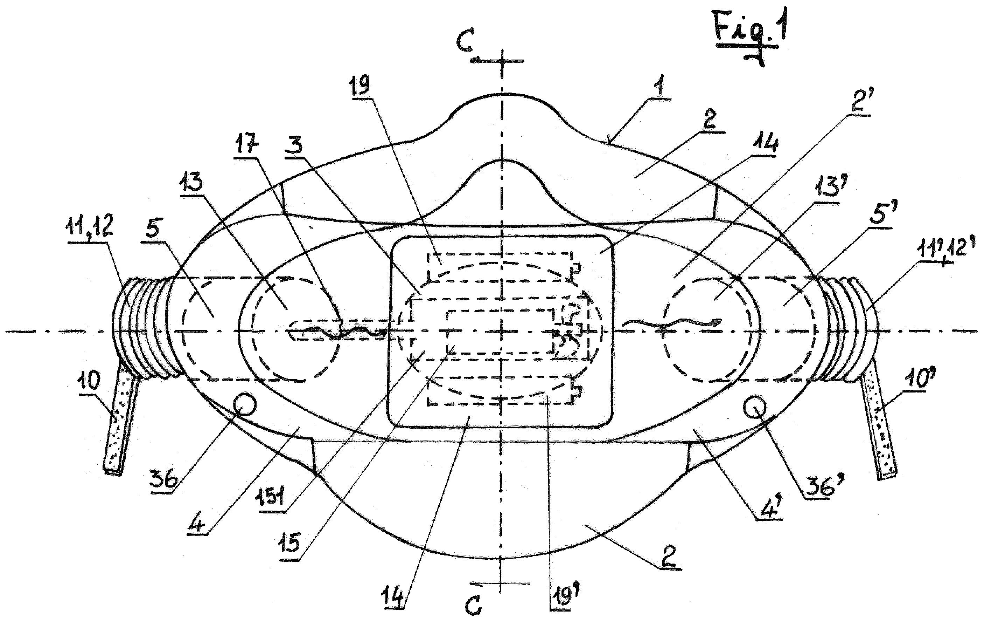

- Figure 1 illustrates a front view from the outside of the device according to this invention;

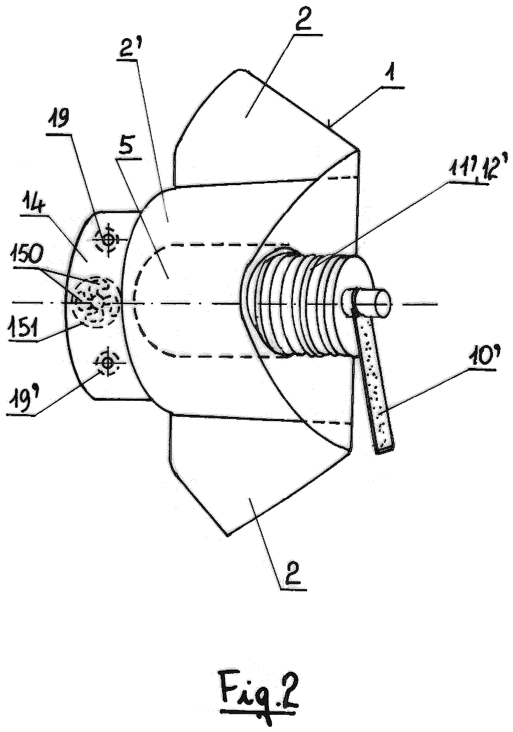

- Figure 2 illustrates a side view of the device of Figure 1;
- Figure 3 illustrates a section according C-C of Figure 1;
- Figure 4 illustrates the sleeve with the spring in the rest position;
- Figure 5 illustrates the sleeve with the spring in the extended position;
- Figure 6 illustrates the spring in the extended position;

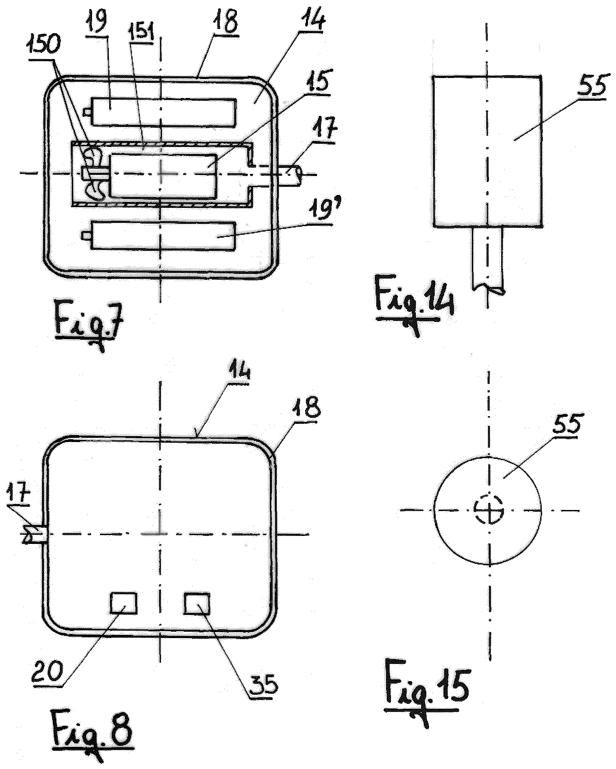

- Figure 7 illustrates the inside front view and partial section of the fan housing box and relative power supply battery;
- Figure 8 illustrates the external front view of the box of Figure 7;
- Figure 9 illustrates an exploded view of the container of the pad in the open position;
- Figure 10 illustrates the section according to A-A of Figure 9;
- Figure 11 illustrates the section according to B-B of Figure 9;
- Figure 12 illustrates the partial section of the sleeve with the container of the pad in the open position;
- Figure 13 illustrates the partial section of the sleeve with the container of the pad in the closed position;
- Figure 14 illustrates the side view of the pad according to the present invention;
- Figure 15 illustrates the top view of the pad of Figure 14;
- Figure 16 illustrates the side view of the pad container closure device;

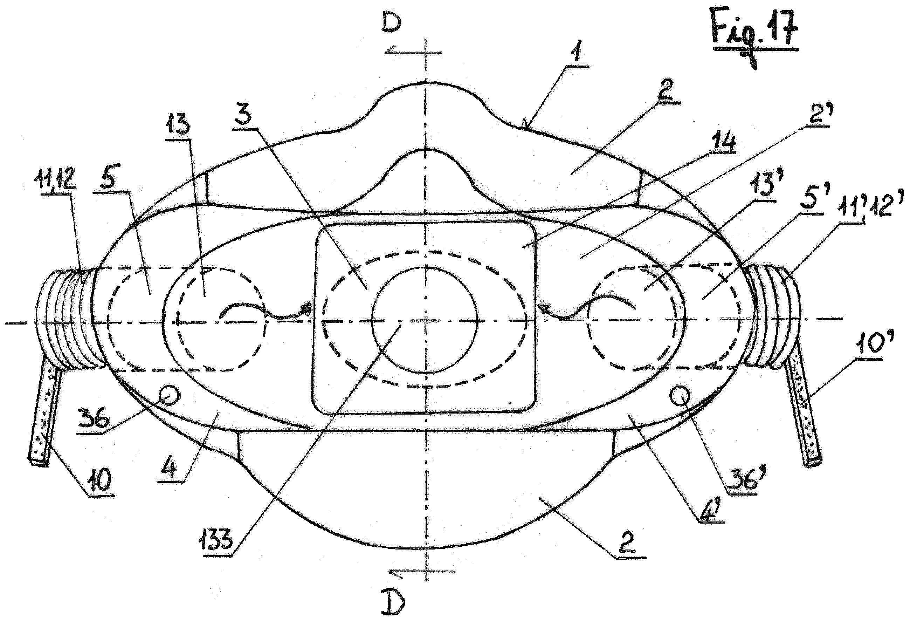

- Figure 17 illustrates a front view from the outside of an alternative embodiment of the respiratory protective device;

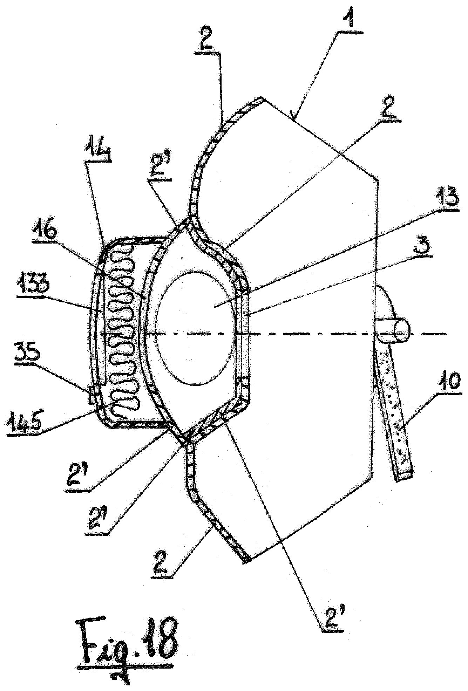

- Figure 18 illustrates the section according to D-D of Figure 17;
- Figure 19 illustrates the enlarged detail E of Figure 13.

With reference to the aforementioned figures, the device according to the present invention is made substantially in the form of a mask and comprises a mask body 1 of any convenient rigid or semi-rigid, soft material, suitable to cover the mouth and nose of a person, preferably formed by a first layer 2 and a second layer 2' substantially superimposed on each other and communicating through at least one hole 3 so as to ensure the passage of air between them. At the sides of the mask body there are at least two housings 4, 4' (one on each side) each designed to hold a respective sleeve 5 or 5', preferably shaped like a cylinder or truncated cone and equipped with an edge 6 in the upper part so as to be able to be joined to said body 1. Said sleeve is advantageously made of a rigid or semi-rigid material.

Each sleeve 5, 5' is provided with at least one pair of fins or other fixing means so as to make it removable from the housing 4, 4'.

Inside each sleeve 5 or 5' a spring 7 or 7' is inserted, preferably in metal or plastic material, provided with a first end 8 or 8' and with a second end 9 or 9' which has fixing means which allow the ends 9, 9' of the two springs to join each other. Such means comprise, for example, a Velcro 10 or 10' and the springs 7 or 7' are capable of extending by several centimeters so as to be hooked each other by means of said Velcro 10 and 10' behind the head of the person wearing the mask.

Each spring 7 or 7' is coated substantially for a first portion (for example, for half of its length) by a cylinder 11 or 11' of non-breathable fabric material and for a second portion by a cylinder 12 or 12' of material in breathable fabric, for example, of non-woven fabric or of material of the type used for FFP1, FFP2 or FFP3 masks or other convenient material capable of filtering the air. Therefore, aeration channels connected to said mask 1 are formed.

In this way, air of higher hygienic quality is introduced, being captured by the back of the person's head and, therefore, less exposed to the dust concentrations and aerosols found in the part in front of the person's face. In addition to this, the wear and tear of the filtering material is reduced with obvious advantages in terms of economics and device reliability.

The air thus obtained is filtered by the material of said cylinder 12 or 12' and circulates freely inside the mask 1.

In an alternative solution, all the cylinders 11, 11', 12 and 12' of the sleeve 5 or 5' are made of breathable material.

Advantageously, a non-return valve 13 or 13' is used, placed in the lower end part of the sleeve 5 or 5', so that the air expelled in the exhalation phase, rich in carbon dioxide, does not remain inside the mask thus allowing the person wearing it to always breathe well-oxygenated air.

In this way, one of the two valves 13 or 13' allows the entry of filtered air, while the other 13' or 13 allows the expelled air to escape in the exhalation phase.

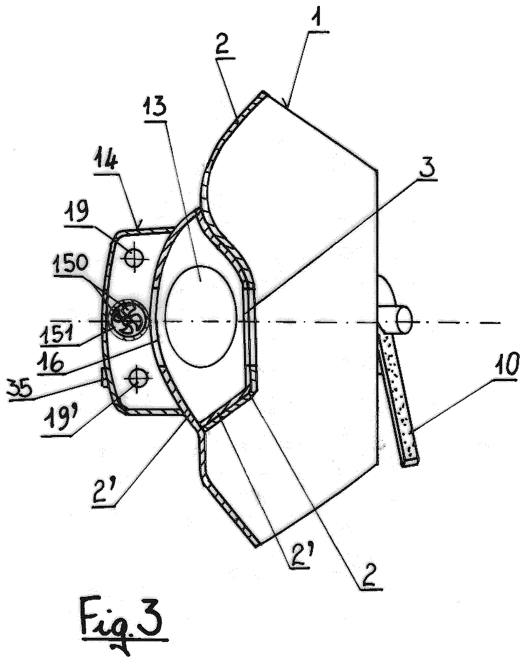

Advantageously, the device, according to the present invention, is equipped with a removable housing box 14, placed in the front part of the mask body 1 and containing a fan 15 which promotes, through a connection hole 16 (Figure 3), the recirculation of the air inside the mask body 1 helping the breathing of the person. The fan 15 includes blades 150 shaped so as to draw inlet air into the mask body 1 from the non-return valve 13 or 13' through a connection tube 17 and promote the escape of the carbon dioxide-rich air from the valve 13' or 13. The fan 15 can be placed inside a cylindrical body 151 joined to the connection tube 17 so as to better convey the incoming air into the mask body 1.

To ensure insulation from the outside, said housing box 14 is equipped with a plastic gasket 18 and is joined to the mask body 1 by means of a snap closure. Said fan 15 operates by means of batteries 19, 19' rechargeable preferably through wireless technology and it can be operated by means of an on / off button 20 placed at the front of said box 14.

The sleeve 5 or 5' is internally equipped, in the central part, with a container 21 which can advantageously have a pad 55. Said container 21 is formed by two male 22 and female 23 parts overlapping each other and provided with side openings 24, 25. The male part 22, to the walls of which the pad 55 is fixed, has an enlarged base 26 preferably in the shape of a truncated cone equipped with a housing seat 27 for the first end of the spring 7 and with an appendix 28 that fits inside the central part of the non-return valve 13. Said spring 7 placed in the sleeve 5' or 5 without the container 21, is fixed to the lower edge of the sleeve itself by means of suitable fastening means.

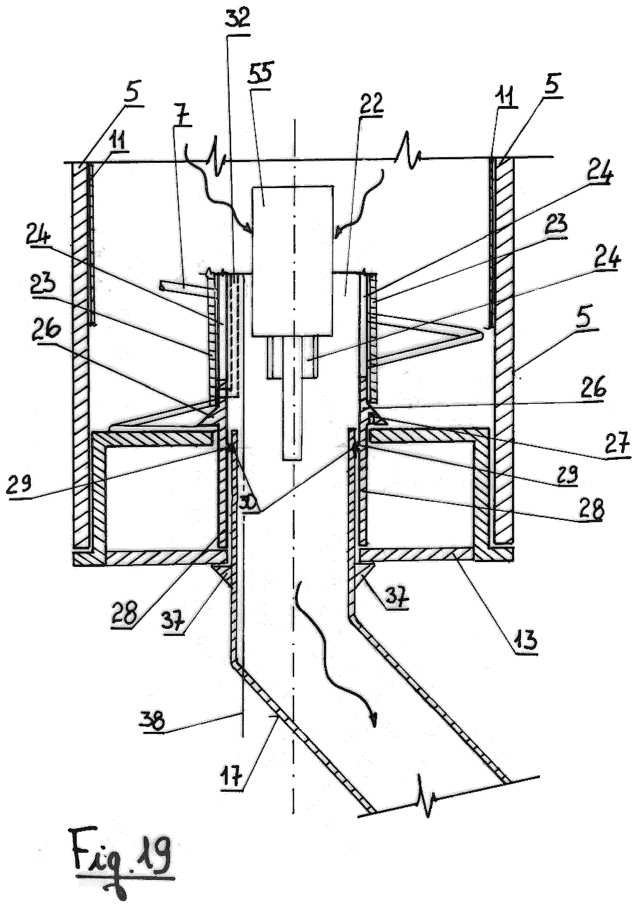

Said appendix 28 is also internally provided with at least one protuberance 29 able to fit into a corresponding cavity 30, as shown in Figure 19, made at the end part of the connection tube 17 which allows the passage of air between the sleeve 5 or 5' and the housing box 14 of the fan 15.

The connection tube 17 has also an enlarged base 37 preferably in a shape of a truncated cone which, when the connection tube 17 is inserted inside said appendix 28, abuts against the external part of the non-return valve 13 blocking it in position.

The container 21 can be closed or opened by rotation R of the female part 23 on the male part 22: in the closed position, the walls of the female part 23 close the respective openings 24 of the male part 22, while in the open position, the respective openings of the two male 22 and female 23 parts overlap each other allowing the passage of air circulating inside the sleeve 5. In order to position and block the aforementioned male and female parts, at least one tooth 31 is provided, on the inner wall of the latter, which fits inside an "L"-shaped groove 32, made on the outer wall of the male part 22 so that, once the tooth 31 has reached the end of the vertical stroke, the female part 23 locks onto the male part 22 by rotation R, remaining in position.

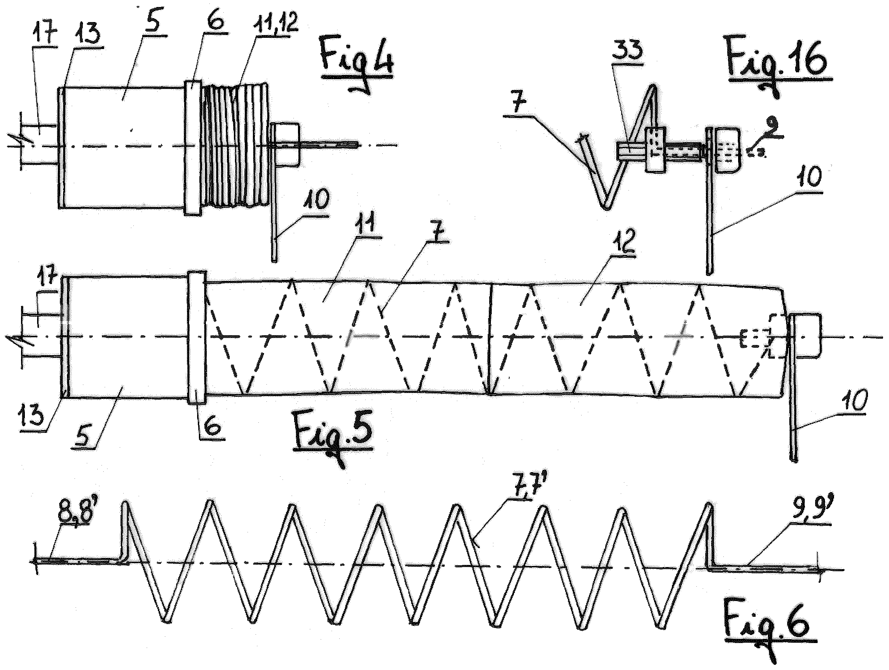

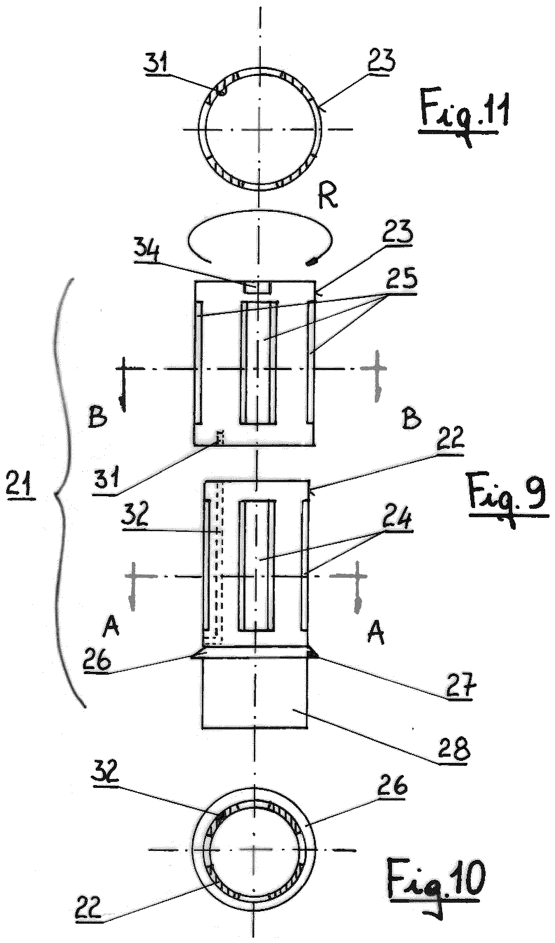

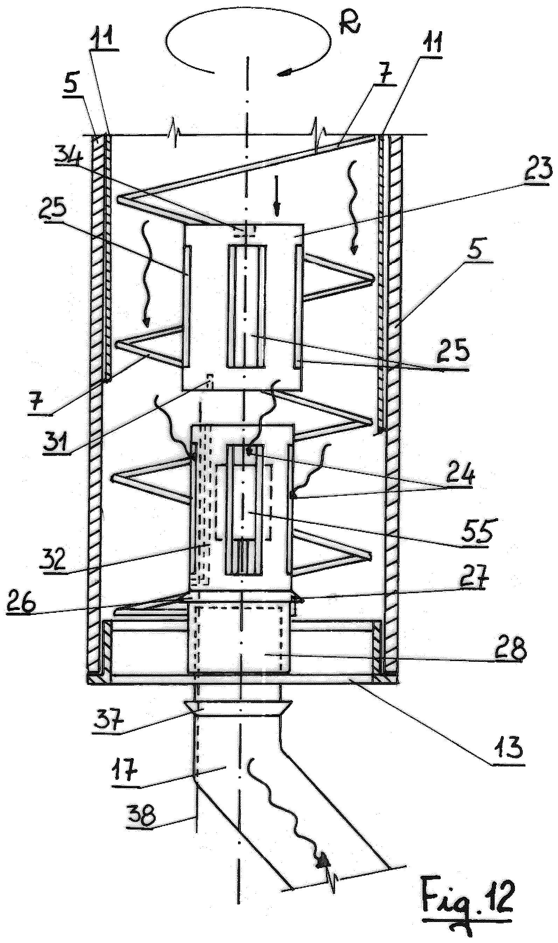

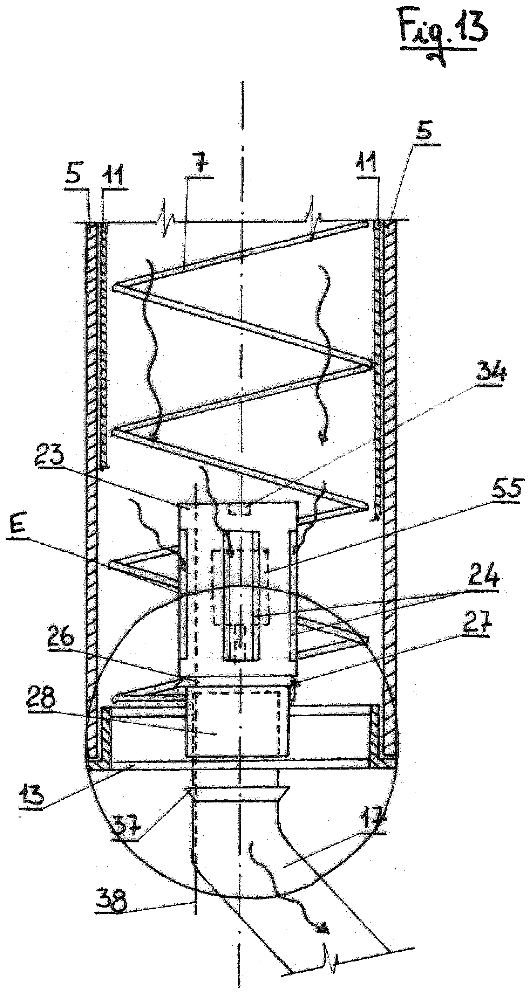

The rotation movement R of the female part on the male is facilitated by the presence of a pin 33 preferably hexagonal (Figure 16) which fits into a specially shaped housing 34 (Figures 9, 12 and 13), preferably hexagonal in shape, placed on the top of the female part 23. A fixing element, for example a Velcro 10, is attached to said pin 33, capable of fixing said sleeve 5, 5' behind the person's head and has inside it a housing seat for the end part of the spring.

In said way, the filtered incoming air is able to reach, through said openings 24, 25, the pad 55 which can be soaked in an aromatic, balsamic or medicinal substance and is able to release it as it passes, so that the aromatized / medicated air is conveyed inside the connection tube 17 to the housing box 14 of the fan 15 and, therefore, inside the mask body 1.

Said pad 55 is preferably activated by heating a resistance 38 placed in the immediate vicinity and / or in contact with it and it can be activated by means of an electro-thermal command 35 placed in the front part of the housing box 14.

In an alternative embodiment, the respiratory protective device provides for air inlet into the mask body 1 from both sleeves 5, 5' and their respective non-return valves 13, 13' while the air outlet takes place via a non-return valve 133 placed in the front of the housing box 14 and a filter 145 made of FFP2, FFP3 material.

Finally, housings 36, 36' placed in the front part of the mask body 1 specially shaped to accommodate bluetooth earphones are provided.

The embodiments described in the present description and the configurations shown in the drawings are only the preferred embodiments of the present invention but the technical variants which fall within the above expressed concept of the present invention are also intended to be included in the scope of the appended claims.

## Claims

1. Protection device for the protection of the respiratory tract of a person comprising:
- a mask body (1) of any convenient rigid or semi-rigid, soft material capable of covering a person's mouth and nose,
- at least one pair of housings (4, 4'), placed on the sides of said mask body (1),
- a pair of sleeves (5, 5'),
wherein the at least one pair of housings (4, 4') is designed to hold said sleeve (5, 5') inside each of them, wherein the front part of the mask body (1) comprises a removable housing box (14) containing a fan (15) which facilitates, by means of a connection hole (16), the recirculation of the air inside the mask body (1), which is sucked by at least one of the two sleeves (5 or 5') through a connection tube (17) placed between them and the housing box (14), by means of a non-return valve (13 or 13') placed in the lower end part of the respective sleeve (5 or 5'),
- two springs (7, 7') fitted with a first end (8, 8') while the second end (9, 9') has fixing means enabling said ends of the two springs (7, 7') to join together behind the head of the person wearing the device, **characterized in that** the first end (8, 8') of each spring (7, 7') is inserted inside each sleeve (5, 5')
wherein each spring (7, 7') is coated substantially for a first portion, the one fixed in the sleeve (5, 5'), by a cylinder (11, 11') of non-breathable fabric material and for, a second portion, by a cylinder (12, 12') of breathable fabric material or other suitable material capable of filtering the air, so that the air introduced into at least one sleeve is captured from the back of the person's head.

2. Protection device according to claim 1, wherein said fan (15) is placed inside a cylindrical body (151) joined to the connection tube (17) so as to better convey the incoming air into the mask body (1).

3. Protection device according to claim 1, wherein said housing box (14) is equipped with a gasket (18) in plastic material and is joined to the mask body (1) by means of a snap closure.

4. Protection device according to claim 1, wherein said said fan (15) works by means of batteries (19, 19') rechargeable through wireless technology and it can be operated by means of an on / off button (20) placed at the front of said box (14).

5. Protection device according to claim 1, wherein the cylinder (12, 12') is made of non-woven fabric or of material of the type used for FFP1, FFP2 or FFP3 masks capable of filtering the air.

6. Protection device according to claim 1, wherein the springs (7, 7') are made of metal or plastic material.

7. Protection device according to claim 1, wherein the mask body (1) is formed by a first layer (2) and a second layer (2') substantially superimposed on each other and communicating through at least one hole (3) so as to guarantee the passage of the air between them.

8. Protection device according to claim 1, wherein each sleeve (5, 5') is provided with at least one pair of fins or other fixing means so as to make it removable from the housing (4, 4').

9. Protection device according to claim 1, wherein the fixing means of the second end (9, 9') of the spring (7, 7') are a strip of Velcro fabric (10, 10') capable of making the hooking and the release at the back of the head of the person wearing the device very quick.

10. Protection device according to claim 1, wherein the mask body (1) is equipped with housings (36, 36') specially shaped to accommodate bluetooth headsets.

11. Protection device according to claim 1, wherein the sleeve (5 or 5') is equipped, internally, in the central part, with a container (21) for a pad (55), said container (21) being formed by two male (22) and female (23) parts overlapping each other and provided with lateral openings (24, 25), in which the male part (22) has an enlarged base (26) preferably in the shape of a truncated cone equipped with a housing seat (27) for the first end (8) of the spring (7), an appendix (28) that fits inside the central part of the non-return valve (13 or 13 ') and a support of the pad (55).

12. Protection device according to claim 11, wherein said appendix (28) is provided, internally, with at least one protuberance (29) able to fit into a corresponding cavity (30) made in the end part of the connection tube (17) which allows the passage of air into the housing box (14) of the fan (15) and is equipped with an enlarged base (37), preferably in a shape of a truncated cone, which abuts against the external part of the non-return valve (13) locking the connection tube (17) in place.

13. Protection device according to claim 11, wherein said container (21) can be closed or opened by rotation R of the female part (23) on the male part (22) and, in the closed position, the walls of the female part (23) close the respective openings (24) of the male part (22), while in the open position, the respective openings (24, 25) of the two male (22) and female (23) parts overlap each other allowing the passage of the air circulating inside the sleeve (5 or 5').

14. Protection device according to claim 11, wherein the female part (23) of the container (21) is equipped, on the inner wall of the latter, with at least one tooth (31), which fits inside an "L"-shaped groove (32), made on the outer wall of the male part (22) so that, when it reaches the end of the vertical stroke and by rotation R of the female part (23), the latter stops and remains in position.

15. Protection device according to claim 13, in which the rotation movement R of the female part (23) on the male (22) is facilitated by the presence of a hexagonal pin (33) which fits into a specially shaped housing (34), placed on the top of the female part (23), wherein a fixing element, for example a Velcro (10), is attached to said pin (33), capable of fixing said sleeve (5, 5') behind the person's head and has inside it a housing seat for the end part of the spring.

16. Protection device according to claim 11, wherein the filtered incoming air is able to reach, through said openings (24, 25), the pad (55) which can be soaked in an aromatic, balsamic or medicinal substance and is able to release it as it passes, so that the aromatized / medicated air is conveyed inside the connection tube (17) to the housing box (14) of the fan (15) and, therefore, inside the mask body (1) .

17. Protection device according to claim 11, wherein said pad (55) is activated by heating a resistance (38) placed in the immediate vicinity and / or in contact with it and it can be activated by means of an electro-thermal command (35) placed in the front part of the housing box (14).

18. Device according to claim 1, wherein the air inlet into the mask body (1) takes place from both sleeves (5, 5') and the respective non-return valves (13, 13') while the air outlet takes place via a non-return valve (133) placed in the front of a housing box (14) and a filter (145) made of FFP1, FFP2 or FFP3 material.

19. Device according to the previous claims, in which the cylinders (11, 11', 12, 12') are made of fabric of breathable material.

## Patentansprüche

1. Schutzvorrichtung zum Schutz der Atemwege einer Person, umfassend:
- einen Maskenkörper (1) aus einem beliebigen geeigneten starren oder halbstarren, weichen Material, das Mund und Nase einer Person abdecken kann,
- mindestens ein Paar von Gehäusen (4, 4'), das an den Seiten des Maskenkörpers (1) platziert ist,
- ein Paar von Hülsen (5, 5'),
wobei das mindestens eine Paar von Gehäusen (4, 4') so ausgestaltet ist, dass es die Hülse (5, 5') jeweils im Inneren hält,
wobei der vordere Teil des Maskenkörpers (1) einen entfernbaren Gehäusekasten (14) umfasst, der einen Lüfter (15) beinhaltet, der mithilfe eines Verbindungslochs (16) die Umwälzung der Luft im Inneren des Maskenkörpers (1) fördert, die von mindestens einer der zwei Hülsen (5 oder 5') durch ein zwischen ihnen und dem Gehäusekasten (14) platziertes Verbindungsrohr (17) angesaugt wird, mithilfe eines Rückschlagventils (13 oder 13'), das im unteren Endteil der jeweiligen Hülse (5 oder 5') platziert ist,
- zwei Federn (7, 7'), die mit einem ersten Ende (8, 8') ausgestattet sind, während das zweite Ende (9, 9') Fixiermittel aufweist, die ermöglichen, die Enden der zwei Federn (7, 7') hinter dem Kopf der Person, die die Vorrichtung trägt, miteinander zu verbinden,
**dadurch gekennzeichnet, dass** das erste Ende (8, 8') jeder Feder (7, 7') im Inneren jeder Hülse (5, 5') eingesetzt ist,
wobei jede Feder (7, 7') im Wesentlichen für einen ersten Abschnitt, den in der Hülse (5, 5') fixierten, durch einen Zylinder (11, 11') aus nicht atmungsaktivem Gewebematerial und, für einen zweiten Abschnitt, durch einen Zylinder (12, 12') aus atmungsaktivem Gewebematerial oder einem anderen geeigneten Material, das die Luft filtern kann, beschichtet ist, sodass die in mindestens eine Hülse eingeführte Luft vom Hinterkopf der Person aufgefangen wird.

2. Schutzvorrichtung nach Anspruch 1, wobei der Lüfter (15) im Inneren eines zylindrischen Körpers (151) platziert ist, der mit dem Verbindungsrohr (17) verbunden ist, um die einströmende Luft besser in den Maskenkörper (1) zu leiten.

3. Schutzvorrichtung nach Anspruch 1, wobei der Gehäusekasten (14) mit einer Dichtung (18) aus Kunststoffmaterial ausgestattet ist und mithilfe eines Schnappverschlusses mit dem Maskenkörper (1) verbunden ist.

4. Schutzvorrichtung nach Anspruch 1, wobei der Lüfter (15) mithilfe von durch Drahtlostechnologie wiederaufladbaren Akkumulatoren (19, 19') arbeitet und mithilfe einer an der Vorderseite des Kastens (14) platzierten Ein-/Aus-Taste (20) betrieben werden kann.

5. Schutzvorrichtung nach Anspruch 1, wobei der Zylinder (12, 12') aus Faservliesstoff oder aus Material des für FFP1-, FFP2- oder FFP3-Masken verwendeten Typs gefertigt ist, der Luft filtern kann.

6. Schutzvorrichtung nach Anspruch 1, wobei die Federn (7, 7') aus Metall oder Kunststoffmaterial gefertigt sind.

7. Schutzvorrichtung nach Anspruch 1, wobei der Maskenkörper (1) durch eine erste Schicht (2) und eine zweite Schicht (2') gebildet ist, die einander im Wesentlichen überlagert sind und durch mindestens ein Loch (3) kommunizieren, um den Durchlass der Luft zwischen ihnen sicherzustellen.

8. Schutzvorrichtung nach Anspruch 1, wobei jede Hülse (5, 5') mit mindestens einem Paar von Rippen oder einem anderen Fixiermittel versehen ist, um sie vom Gehäuse (4, 4') abnehmbar zu machen.

9. Schutzvorrichtung nach Anspruch 1, wobei die Fixiermittel des zweiten Endes (9, 9') der Feder (7, 7') ein Streifen aus Velcro-Gewebe (10, 10') sind, das das Einhaken und die Freigabe am Hinterkopf der Person, die die Vorrichtung trägt, sehr rasch machen kann.

10. Schutzvorrichtung nach Anspruch 1, wobei der Maskenkörper (1) mit Gehäusen (36, 36') ausgestattet ist, die besonders geformt sind, um Bluetooth-Hörsprechgarnituren aufzunehmen.

11. Schutzvorrichtung nach Anspruch 1, wobei die Hülse (5 oder 5') intern im zentralen Teil mit einem Behälter (21) für ein Pad (55) ausgestattet ist, wobei der Behälter (21) durch zwei Steck- (22) und Buchsenteile (23) gebildet ist, die einander überlappen und mit seitlichen Öffnungen (24, 25) versehen sind, wobei der Steckteil (22) eine vergrößerte Basis (26), vorzugsweise in Form eines Kegelstumpfes, der mit einem Gehäusesitz (27) für das erste Ende (8) der Feder (7), einen Fortsatz (28), der ins Innere des Mittelteils des Rückschlagventils (13 oder 13') passt, und einen Träger für das Pad (55) aufweist.

12. Schutzvorrichtung nach Anspruch 11, wobei der Fortsatz (28) intern mit mindestens einer Protuberanz (29) versehen ist, die in einen entsprechenden Hohlraum (30) passt, der im Endteil des Verbindungsrohrs (17) gebildet ist, der den Durchlass von Luft in den Gehäusekasten (14) des Lüfters (15) erlaubt und mit einer vergrößerten Basis (37) ausgestattet ist, vorzugsweise in Form eines Kegelstumpfes, der an den externen Teil des Rückschlagventils (13) stößt, was das Verbindungsrohr (17) an Ort und Stelle sichert.

13. Schutzvorrichtung nach Anspruch 11, wobei der Behälter (21) durch eine Drehung R des Buchsenteils (23) am Steckteil (22) geschlossen oder geöffnet werden kann und die Wände des Buchsenteils (23) in der geschlossenen Position die jeweiligen Öffnungen (24) des Steckteils (22) schließen, während die jeweiligen Öffnungen (24, 25) der beiden Steck- (22) und Buchsenteile (23) einander in der offenen Position überlappen, was den Durchlass der Luft erlaubt, die im Inneren der Hülse (5 oder 5') zirkuliert.

14. Schutzvorrichtung nach Anspruch 11, wobei der Buchsenteil (23) des Behälters (21) an der Innenwand des Letzteren mit mindestens einem Zahn (31) versehen ist, der ins Innere einer "L"-förmigen Nut (32) passt, die an der Außenwand des Steckteils (22) gefertigt ist, sodass Letzterer, wenn er das Ende des vertikalen Hubs erreicht, und durch Drehung R des Buchsenteils (23), anhält und in Position bleibt.

15. Schutzvorrichtung nach Anspruch 13, wobei die Drehbewegung R des Buchsenteils (23) am Stecker (22) durch das Vorhandensein eines hexagonalen Stifts (33) gefördert wird, der in ein besonders geformtes Gehäuse (34) passt, oben auf dem Buchsenteil (23) platziert, wobei ein Fixierelement, zum Beispiel ein Velcro (10), am Stift (33) befestigt ist, das die Hülse (5, 5') hinter dem Kopf der Person fixieren kann und in seinem Inneren einen Gehäusesitz für den Endteil der Feder aufweist.

16. Schutzvorrichtung nach Anspruch 11, wobei die gefilterte einströmende Luft durch die Öffnungen (24, 25) das Pad (55) erreichen kann, das in einer aromatischen, balsamischen oder medizinalen Substanz getränkt werden kann und diese während des Durchlaufens freigeben kann, sodass die aromatisierte/heilkräftige Luft im Inneren des Verbindungsrohrs (17) zum Gehäusekasten (14) des Lüfters (15) und deshalb im Inneren des Maskenkörpers (1) befördert wird.

17. Schutzvorrichtung nach Anspruch 11, wobei das Pad (55) durch Erwärmen eines Widerstands (38) aktiviert wird, der in unmittelbarer Nähe und/oder in Kontakt mit diesem platziert ist, und mithilfe eines elektrothermischen Befehls (35) aktiviert werden kann, der im vorderen Teil des Gehäusekastens (14) platziert ist.

18. Vorrichtung nach Anspruch 1, wobei der Lufteinlass in den Maskenkörper (1) von beiden Hülsen (5, 5') und den jeweiligen Rückschlagventilen (13, 13') erfolgt, während der Luftauslass über ein Rückschlagventil (133), das an der Vorderseite eines Gehäusekastens (14) platziert ist, und ein aus FFP1-, FFP2- oder FFP3-Material gefertigtes Filter (145) erfolgt.

19. Vorrichtung nach den vorhergehenden Ansprüchen, wobei die Zylinder (11, 11', 12, 12') aus Gewebe aus atmungsaktivem Material gefertigt sind.

## Revendications

1. Dispositif de protection pour la protection des voies respiratoires d'une personne comprenant :
- un corps de masque (1) de tout matériau approprié rigide ou semi-rigide, souple, capable de recouvrir la bouche et le nez d'une personne,
- au moins une paire de logements (4, 4'), placés sur les côtés dudit corps de masque (1),
- une paire de manchons (5, 5'),
dans lequel la au moins une paire de logements (4, 4') est conçue pour maintenir ledit manchon (5, 5') à l'intérieur de chacun d'eux,
dans lequel la partie avant du corps de masque (1) comprend un boîtier de logement amovible (14) contenant un ventilateur (15) qui facilite, au moyen d'un trou de connexion (16), la recirculation de l'air à l'intérieur du corps de masque (1), qui est aspiré par au moins l'un des deux manchons (5 ou 5') à travers un tube de connexion (17) placé entre eux et le boîtier (14) de logement, au moyen d'un clapet anti-retour (13 ou 13') placé dans la partie d'extrémité inférieure du respectif manchon (5 ou 5'),
- deux ressorts (7, 7') munis d'une première extrémité (8, 8') tandis que la seconde extrémité (9, 9') présente des moyens de fixation permettant auxdites extrémités des deux ressorts (7, 7') de se joindre derrière la tête de la personne portant le dispositif,
**caractérisé en ce que** la première extrémité (8, 8') de chaque ressort (7, 7') est insérée à l'intérieur de chaque manchon (5, 5')
dans lequel chaque ressort (7, 7') est revêtu substantiellement pour une première portion, celle fixée dans le manchon (5, 5'), par un cylindre (11, 11') de matériau de tissu non transpirant et pour une seconde portion, par un cylindre (12, 12') de matériau de tissu transpirant ou d'un autre matériau approprié capable de filtrer l'air, de sorte que l'air introduit dans au moins un manchon est capturé à partir de la partie arrière de la tête de la personne.

2. Dispositif de protection selon la revendication 1, dans lequel ledit ventilateur (15) est placé à l'intérieur d'un corps cylindrique (151) joint au tube de connexion (17) de manière à mieux acheminer l'air entrant dans le corps de masque (1).

3. Dispositif de protection selon la revendication 1, dans lequel ledit boîtier (14) de logement est équipé d'une garniture (18) en matière plastique et est jointe au corps de masque (1) au moyen d'une fermeture à pression.

4. Dispositif de protection selon la revendication 1, dans lequel ledit ventilateur (15) fonctionne au moyen de batteries (19, 19') rechargeables par technologie sans fil et il peut être actionné au moyen d'un bouton on/off (20) placé à l'avant dudit boîtier (14).

5. Dispositif de protection selon la revendication 1, dans lequel le cylindre (12, 12') est réalisé de tissu non-tissé ou d'un matériau du type utilisé pour les masques FFP1, FFP2 ou FFP3 aptes à filtrer l'air.

6. Dispositif de protection selon la revendication 1, dans lequel les ressorts (7, 7') sont réalisés de métal ou de matière plastique.

7. Dispositif de protection selon la revendication 1, dans lequel le corps de masque (1) est formé par une première couche (2) et une seconde couche (2') substantiellement superposées l'une sur l'autre et communiquant à travers au moins un trou (3) de manière à garantir le passage de l'air entre elles.

8. Dispositif de protection selon la revendication 1, dans lequel chaque manchon (5, 5') est pourvu d'au moins une paire d'ailettes ou d'autres moyens de fixation de manière à le rendre amovible du logement (4, 4').

9. Dispositif de protection selon la revendication 1, dans lequel les moyens de fixation de la seconde extrémité (9, 9') du ressort (7, 7') sont une bande de tissu Velcro (10, 10') apte à réaliser l'accrochage et la libération derrière la tête de la personne portant le dispositif très rapidement.

10. Dispositif de protection selon la revendication 1, dans lequel le corps de masque (1) est équipé de logements (36, 36') spécialement formés pour accueillir des casques Bluetooth.

11. Dispositif de protection selon la revendication 1, dans lequel le manchon (5 ou 5') est équipé, intérieurement, dans la partie centrale, d'un récipient (21) pour un tampon (55), ledit récipient (21) étant formé de deux parties mâle (22) et femelle (23) se chevauchant et munies d'ouvertures latérales (24, 25), dans lequel la partie mâle (22) a une base élargie (26) de préférence en forme de tronc de cône équipée d'un siège de logement (27) pour la première extrémité (8) du ressort (7), d'un appendice (28) qui s'adapte à l'intérieur de la partie centrale du clapet anti-retour (13 ou 13') et d'un support du tampon (55).

12. Dispositif de protection selon la revendication 11, dans lequel ledit appendice (28) est pourvu, à l'intérieur, d'au moins une protubérance (29) apte à s'adapter dans une cavité (30) correspondante réalisée dans la partie d'extrémité du tube de connexion (17) qui permet le passage de l'air dans le boîtier (14) de logement du ventilateur (15) et est équipé d'une base élargie (37), de préférence en forme de tronc de cône, qui vient en butée contre la partie externe du clapet anti-retour (13) en bloquant le tube de connexion (17) en place.

13. Dispositif de protection selon la revendication 11, dans lequel ledit récipient (21) peut être fermé ou ouvert par rotation R de la partie femelle (23) sur la partie mâle (22) et, dans la position fermée, les parois de la partie femelle (23) ferment les respectives ouvertures (24) de la partie mâle (22), tandis que dans la position ouverte, les respectives ouvertures (24, 25) des deux parties mâle (22) et femelle (23) se chevauchent permettant le passage de l'air circulant à l'intérieur du manchon (5 ou 5').

14. Dispositif de protection selon la revendication 11, dans lequel la partie femelle (23) du récipient (21) est équipée, sur la paroi interne de ce dernier, d'au moins une dent (31), qui s'adapte à l'intérieur d'une rainure (32) en forme de « L », ménagée sur la paroi externe de la partie mâle (22) de sorte que, lorsqu'elle atteint la fin de la course verticale et par rotation R de la partie femelle (23), cette dernière s'arrête et reste en position.

15. Dispositif de protection selon la revendication 13, dans lequel le mouvement de rotation R de la partie femelle (23) sur la partie mâle (22) est facilité par la présence d'une broche (33) hexagonale qui s'adapte dans un logement (34) de forme spéciale, placé sur le dessus de la partie femelle (23), dans lequel un élément de fixation, par exemple un Velcro (10), est fixé à ladite broche (33), capable de fixer ledit manchon (5, 5') derrière la tête de la personne et a à l'intérieur un siège de logement pour la partie d'extrémité du ressort.

16. Dispositif de protection selon la revendication 11, dans lequel l'air entrant filtré est capable d'atteindre, à travers lesdites ouvertures (24, 25), le tampon (55) qui peut être imbibé d'une substance aromatique, balsamique ou médicinale et est capable de la libérer lors de son passage, de sorte que l'air aromatisé/médicamenteux est acheminé à l'intérieur du tube de connexion (17) vers le boîtier (14) de logement du ventilateur (15) et, par conséquent, à l'intérieur du corps de masque (1).

17. Dispositif de protection selon la revendication 11, dans lequel ledit tampon (55) est activé par chauffage d'une résistance (38) placée à proximité immédiate et/ou en contact avec lui et il peut être activé au moyen d'une commande (35) électrothermique placée dans la partie avant du boîtier (14) de logement.

18. Dispositif selon la revendication 1, dans lequel l'entrée d'air dans le corps de masque (1) a lieu à partir des deux manchons (5, 5') et des respectifs clapets anti-retour (13, 13') tandis que la sortie d'air a lieu via un clapet anti-retour (133) placé à l'avant d'un boîtier (14) de logement et un filtre (145) en matériau FFP1, FFP2 ou FFP3.

19. Dispositif selon les revendications précédentes, dans lequel les cylindres (11, 11', 12, 12') sont réalisés en tissu de matière transpirante.
